# EUROPEAN PATENT SPECIFICATION

(11) **EP 3 423 115 B1**
(45) Date of publication and mention of the grant of the patent: **12.10.2022**
(21) Application number: 17716994.3
(22) Date of filing: 03.03.2017
(51) Int. Cl.: A61L 2/10, C02F 1/32

(54) **APPARATUS FOR OPAQUE FLUID DISINFECTION WITH ULTRAVIOLET EMISSION**
VORRICHTUNG ZUR OPAKEN FLÜSSIGKEITSDESINFEKTION MIT ULTRAVIOLETTER EMISSION
APPAREIL POUR LA DÉSINFECTION DE FLUIDE OPAQUE PAR ÉMISSION D'ULTRAVIOLETS

(30) Priority: 03.03.2016 IT UA20161331
(43) Date of publication of application: 09.01.2019
(73) Proprietor: Margi S.r.l., 43126 Parma (IT); FMB Engineering Innovation For Enterprise S.r.l., 43124 Parma (IT)
(72) Inventor: GUALAZZI, Mario, 43126 Parma (IT); MONTANARI, Roberto, 43124 Parma (IT); SOLARI, Federico, 43126 Parma (IT); DELSANTE, Luigi, 43126 Parma (IT); BARBIERI, Daniele, 43126 Parma (IT)
(74) Representative: Dallaglio, Fabrizio
(86) International application number: PCT/IB2017/051242
(87) International publication number: WO 2017/149501

(56) References cited:
- EP-A1- 1 882 521
- EP-A2- 2 792 371
- WO-A1-92/22502
- WO-A1-2011/014944
- WO-A1-2011/026619
- US-A- 5 874 740

## Description

### FIELD OF APPLICATION OF THE INVENTION

The present finding is inserted in the field of processes and systems for treating opaque fluids, fluids with low transmittance. It is briefly recalled that transmittance, in optics and spectroscopy, is the fraction of light incident at a given wavelength that crosses a sample.

The invention is an apparatus and a method for the sanification/sterilization of opaque fluids by means of ultraviolet radiation; in other words, the invention is set to abate the bacterial load also in those opaque fluids that are hard to treat with UV rays, due to the fact that they have low values of transmittance, where with "low values of transmittance" it is intended fluids having transmittance even lower than 1%.

In particular, the treatment method has effective use in the reduction of the bacterial load of milk whey, i.e. the liquid part of milk that is separated from the curd during cheese-making.

### STATE OF THE ART

WO9222502 discloses a reactor including a UV radiation lamp. The lamp is positioned in the reactor within a transparent sheath, wherein a plurality of baffles are secured to the inside surface of the outer wall, each baffle is circular shaped with a central opening which provides clearance for the protective sheath as well as the system generally designated for cleaning the protective sheath. The baffles are provided within the annular chamber to induce turbulence in the flow to ensure adequate mixing so that during the residence time for the aqueous media all portions thereof are exposed to the UV radiation from lamp to ensure the desired treatment for the aqueous media.

The laws relative to industrial production, the increased general interest and the evolution of strains of resistant bacteria all necessitate an effective application of alternative treatment/disinfection methods, such as treatment with UV.

However, opaque fluids render the currently-known treatments and the action of UV lamps little effective.

One example of an apparatus for treatment by means of UV is described in US 5,874,740. In such document, an apparatus is described in which a plurality of UV tubes are provided with a scraper ring for cleaning the tubes themselves. The scraper rings are in contact with the surface of the tubes due to the presence of O-ring gaskets, and at their interior they define a chamber into which phosphoric acid is injected for cleaning the tube.

Other examples of similar apparatuses are described: WO 2011/014944, EP 2 792 371, WO 2011/026619 and EP 1 882 521.

### DESCRIPTION AND ADVANTAGES OF THE FINDING

One object of the present invention is to provide the art with an apparatus for treating opaque fluids in the scope of a simple, rational solution with rather limited cost.

These and other objects are attained due to the characteristics of the invention reported in independent claim 1. The dependent claims delineate preferred and/or particularly advantageous aspects of the invention.

In accordance with the aforesaid objects, the invention refers to a system for conveying the fluid to be sanified, which ensures the maximum performance and effectiveness of treatment for the fluid to be treated.

In one formulation of the present finding, the apparatus for opaque fluid disinfection with ultraviolet emission can comprise at least one reactor chamber, within which an ultraviolet light source is protected inside a quartz tube. At least one portion of the quartz tube is coaxially inserted in a jacket of larger size, and the fluid to be treated passes into the space defined between said tube of larger size and said quartz tube.

Due to this solution, a sanifying bactericide effect is ensured, by acting on a thin and continuous annular flow of opaque fluid.

In addition, and in accordance with one characteristic of the present invention, the above-indicated treatment device is part of a set equipment piece which provides for means for adhering the tubes and the interior of the chamber of the reactor, such means acting as a mechanical cleaning system.

Due to this solution, it is possible to remove, only with the movement of the movable treatment group, the deposits/residues from the quartz tubes, avoiding manual cleaning.

In addition, this always ensures the maximum UV transmittance of the plant.

As stated, the invention also claims the process of treating milk whey adapted to obtain a bactericide effect, by creating a thin and continuous annular flow of opaque fluid, supplying the UV radiation from within said created annular flow. The separation means between UV irradiation source and the thin and continuous flow of opaque fluid is constituted by quartz.

Due to this solution, significant abatement of the bacterial load was verified, even at high flow rate levels. In the following table, the abatements measured on enterobacteria and total bacterial load at 30°C are recorded.

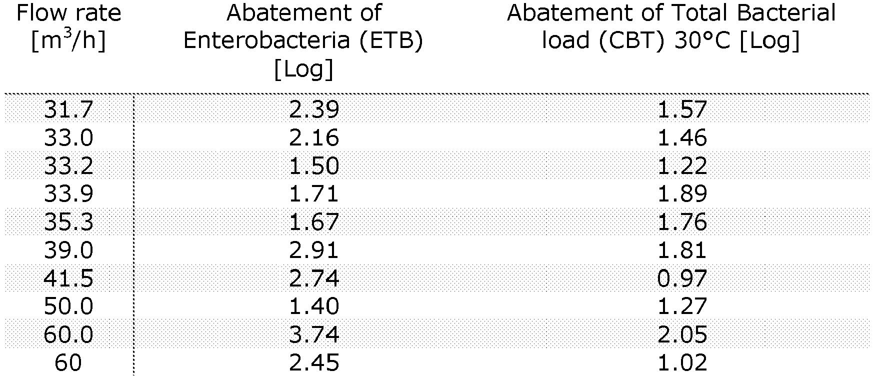

The thickness of the annular passage, i.e. of the annular interspace, is preferably in the interval comprised between 3 and 6 millimeters, preferably 4 or 5 millimeters.

Finally, the invention is set to create a "self-managed" cleaning system by means of the reciprocating translation of the means adapted to create the thin annular flow and providing for scraping rings.

Said objects and advantages are all attained by the apparatus for opaque fluid disinfection with ultraviolet emission, object of the present invention, which is characterized for that provided in the below-reported claims.

### BRIEF DESCRIPTION OF THE FIGURES

This and other characteristics will be clearer from the following description of several embodiments illustrated, merely by way of a non-limiting example, in the enclosed drawing tables.

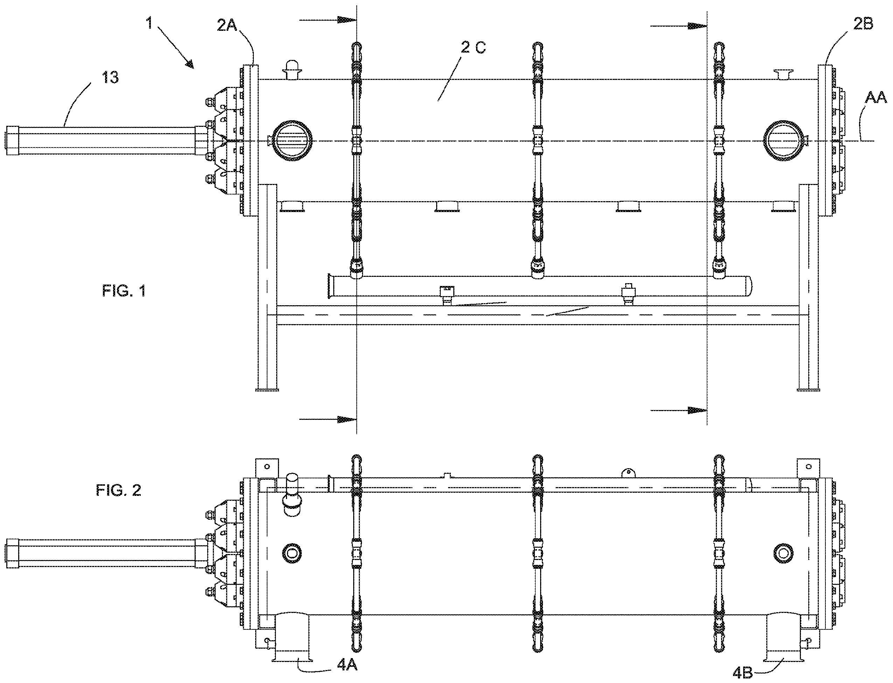

- Figures 1 and 2: illustrate an external view of the apparatus constituted by a reactor chamber,

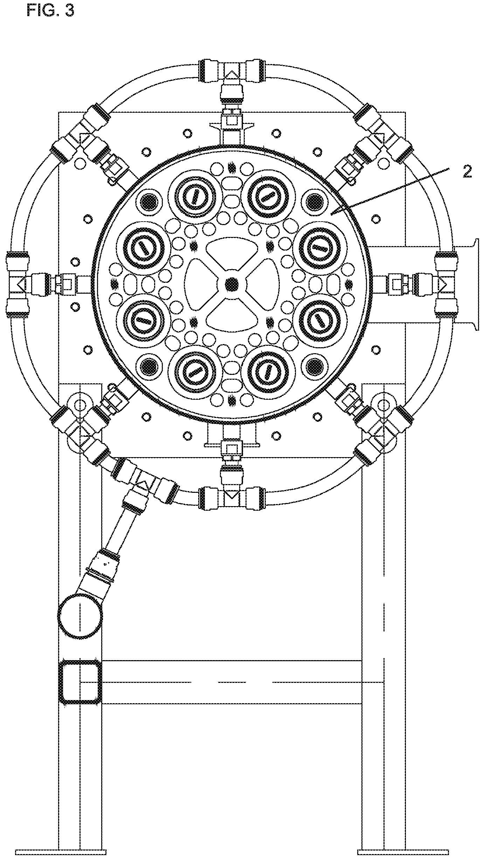

- Figures 3 and 4: illustrate two views, of which one is sectioned with detail of the washing system with pressurized water,

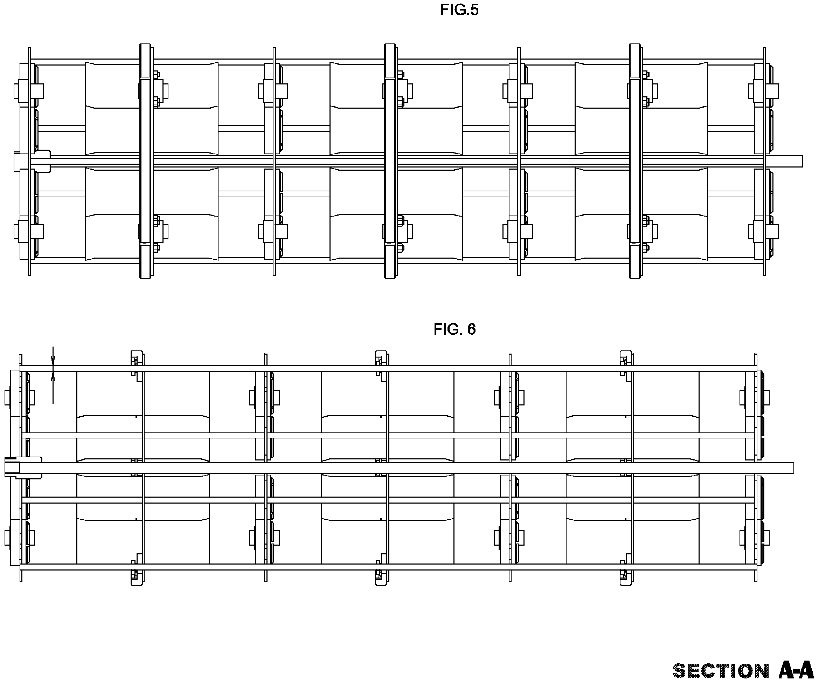

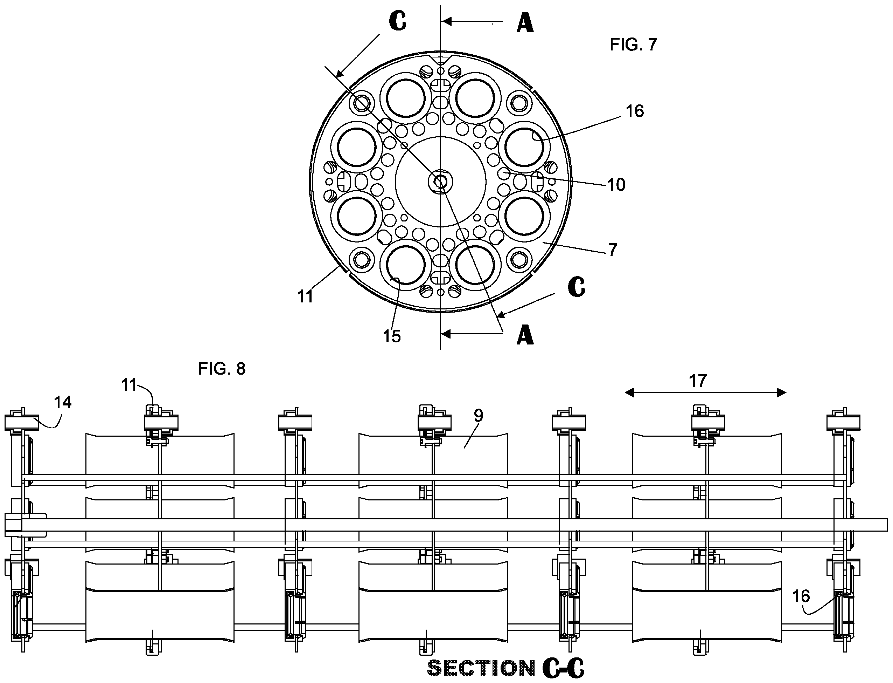

- Figures 5, 6, 7, 8: illustrate four views of the movable group,
- Figure 9: illustrates only the movable group in an axonometric view thereof,
- Figure 10: illustrates only one treatment chamber in detail,

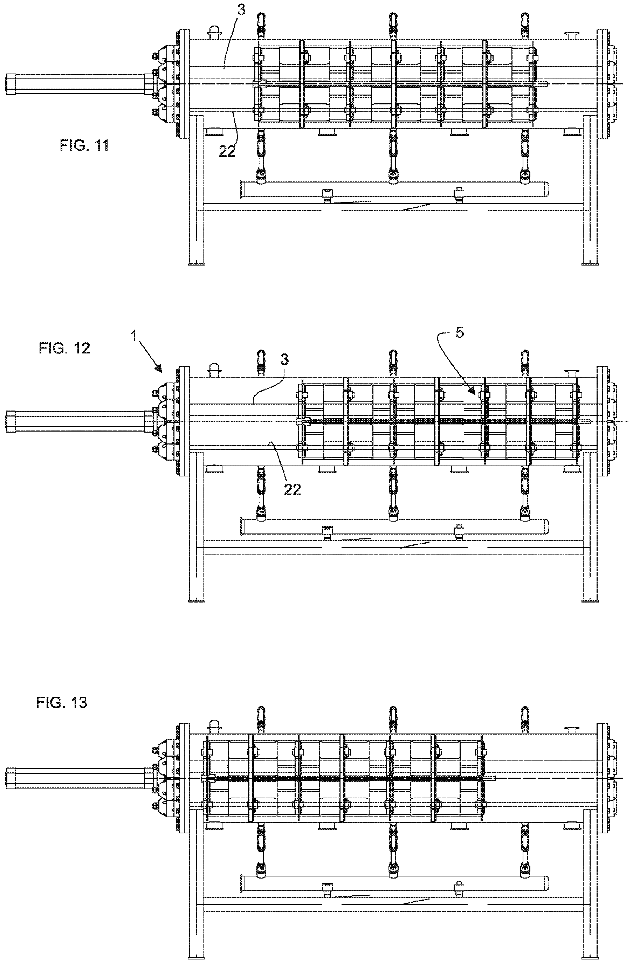

- Figures 11, 12, 13: illustrate the apparatus with the movable group therein and positioned according to three possible configurations thereof, wherein the first configuration corresponds to the normal treatment position on the opaque fluid while the other two configurations depict the right and left limit positions given by the reciprocating movement in order to self-induce the cleaning of the tubes by means of respective scraping rings.

### DESCRIPTION OF THE FINDING

With particular reference to the above-indicated figures, reference number 1 overall indicates an apparatus for treating opaque fluids according to the invention.

The apparatus 1 comprises a reactor body 2C in which a reactor chamber 2 is defined, in more detail a hollow cylindrical body that is closed at the ends 2A, 2B. Preferably, the cylindrical reactor chamber 2 is closed at the longitudinal ends 2A, 2B by a first and a second terminal respectively provided with an inlet port 4A from which a fluid to be treated enters and with an outlet port 4B) from which the treated fluid exits from the reactor chamber 2.

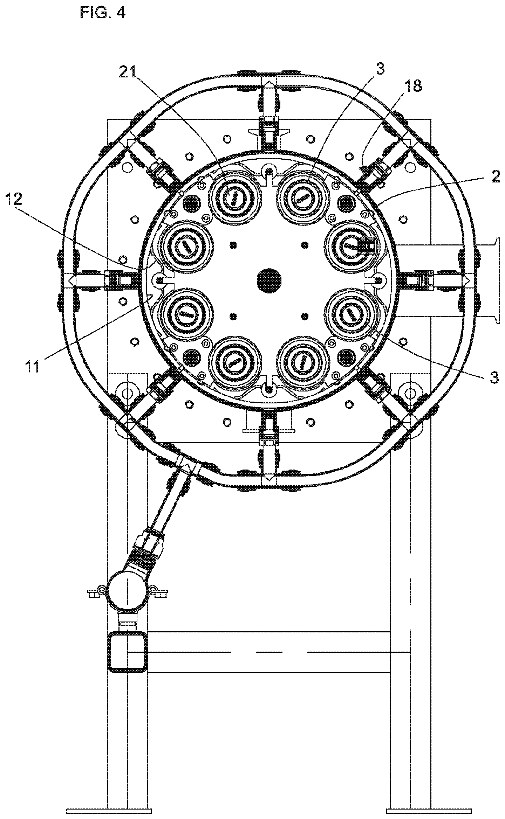

Said reactor chamber 2 comprises a tube bundle therein (FIG. 4) constituted by tubes 3, preferably made of quartz, in turn containing a germicidal lamp 21 UV. Therefore, the group of tubes 3 is fixed and longitudinally arranged inside the reactor body; analogously, also the lamps 21 are fixed.

The reactor body 2 therefore receives the fluid to be treated through an inlet port 4A; the fluid, after having been treated as described hereinbelow, exits from the reactor body 2 from the outlet port 4B.

For the sake of simplicity, AA indicates the axis of the cylindrical reactor body 2.

The series of quartz tubes 3 are at a certain distance from each other; specifically, with respect to the cross section, eight tubes are observed, and it follows that they are at 45° with respect to each other arranged along the entire circular section of the reactor chamber 2; nevertheless, it is specified that the protection is not limited by the number or size or power of the lamps 21 and tubes 3. In one embodiment, the tubes are uniformly distributed around the longitudinal extension axis of the reactor chamber 2.

Returning to the description, it is recalled that the UV lamps 21 are situated within each quartz tube; the fluid however (which is opaque, with low transmittance, and preferably milk whey) passes inside the chamber 2 and externally with respect to the tubes 3.

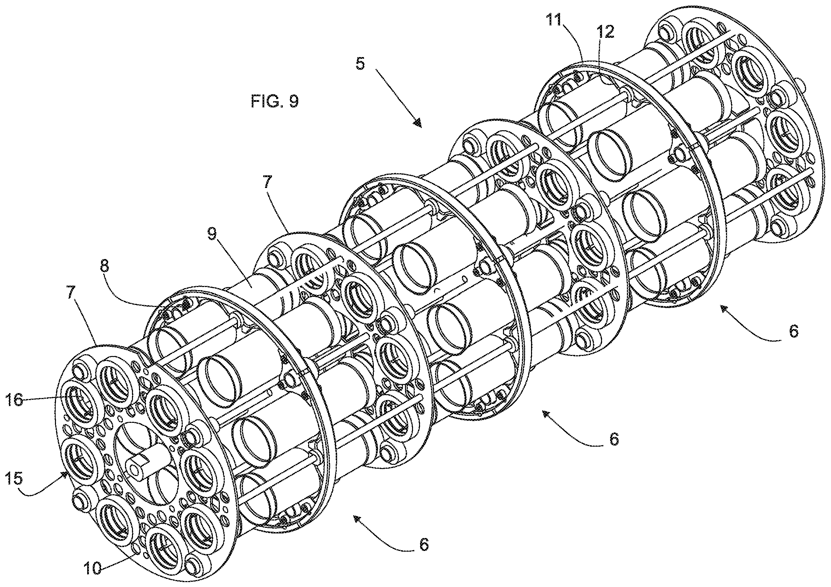

The reactor chamber 2 is equipped with a movable scraper 5, also defined hereinbelow as a movable group (fig. 9). According to one aspect of the invention, the movable scraper 5 is constituted by a plurality of sub-chambers 6, also defined as treatment chambers, arranged at the interior thereof. Even if hereinbelow a movable group 5 is described with a plurality of sub-chambers 6 associated therewith, the invention can also be intended as already defined with a single sub-chamber (hence the movable group 5 can be a single sub-chamber 6).

Continuing with more detail, the movable scraper 5 can be extended for an overall length smaller than the internal length of one or more quartz tubes, i.e. than the interior of the reactor chamber 2.

According to one aspect of the invention, the movable scraper 5 comprises at least one pair of cleaning baffles 7. Preferably, a pair of cleaning baffles 7 delimits a sub-chamber 6 in the reactor chamber 2.

The sub-chamber 6 can identify a treatment area 17, better described hereinbelow, whose length L is smaller than the length L1 of the treatment chamber 6.

In more detail, the sub-chambers 6 are defined (and divided from each other) by a pair of cleaning baffles 7, laterally arranged with respect to the treatment area (i.e. upstream and downstream with respect to the fluid flow). It follows that the length of the treatment area 17 is smaller than the distance between said cleaning baffles 7.

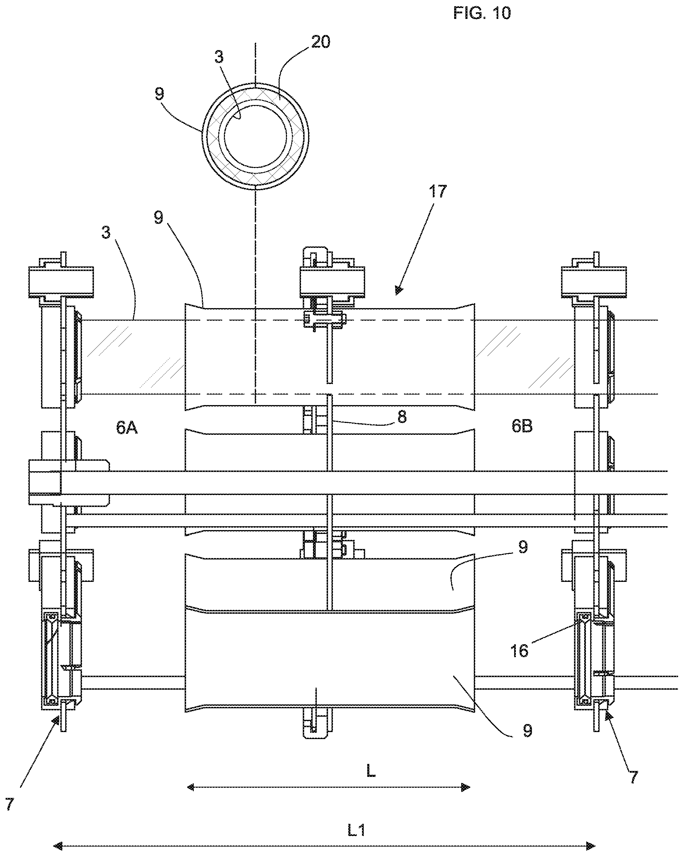

In general and as indicated in the enclosed tables, in order to optimize the treatment process, a multiplicity of sub-chambers 6 are present inside the reactor chamber 2; in turn such sub-chambers are defined by at least one treatment area 17 (fig. 9 and fig. 10). Therefore, the sub-chambers 6, as illustrated, are connected to each other and divided by the respective cleaning baffles 7 so as to preferably form the movable scraper 5: in the embodiment, one observes four cleaning baffles 7, two at the ends and two central baffles, for operating in three sub-chambers 6.

According to one aspect of the invention, the apparatus comprises a plurality of tube holder baffles 8, each tube holder baffle 8 supporting a respective tubular jacket 9 and dividing a respective sub-chamber 6 delimitated by a pair of cleaning baffles 7.

The longitudinal extension L of the jacket 9 can be smaller than the distance L1 between a pair of cleaning baffles defining the sub-chamber space 6.

According to one aspect of the invention, each sub-chamber 6 can also comprises, at its interior, a further baffle that is indicated as tube holder baffle 8 and respective conveyance jackets 9.

In other words, the apparatus 1 can comprise, inside the reactor chamber 2, a tube holder baffle and a tubular jacket 9 supported by the tube holder baffle 8. The movable scraper 5 can comprise the tube holder baffle 8.

The baffle 8 is substantially a discoid element, arranged transversely to the axis AA of the chamber 2; in addition, it has size substantially equal to the interior of the reactor chamber 2, as better specified hereinbelow.

Also the cleaning baffles 7 can be discoid elements arranged as the tube holder baffles 8.

Each tube holder baffle 8 is adapted to divide, into two parts 6A, 6B, the relative treatment area 17 and hence also the treatment chamber 6.

Each tube holder baffle 8 is also adapted to support the series of tubular jackets 9; in such a manner, each jacket 9, which has size greater than that of the quartz tubes 3, can be maintained coaxial with the respective quartz tube 3 such that the difference of diameter between said elements 3 and 9 comes to define an annular interspace 20, of thin thickness, within which the fluid to be treated passes. From this, there is the definition of thin film thickness as intended by the present invention.

In such a manner, the times of effective treatment of the opaque fluid with the UV light source is limited to the passage inside the interspace created between the external jacket 9 (also described as tube hereinbelow) and the fixed quartz tube 3.

In other words, the annular interspace 20 between the tube 3 and the jacket 9 allows the passage of the fluid to be treated through the tube holder baffle 8.

The opaque fluid that enters into the reactor body 2 is introduced into the treatment chamber 6, i.e. it passes from one treatment chamber to the other, through a series of holes 10 made on the cleaning baffles 7.

The fluid, once it has entered into the first section 6A of the treatment chamber 6, is forced by the tube holder baffle 8 to travel along one of the interspaces 20 before then passing into the other half 6B of the treatment chamber, and exits from the holes 10 of the opposite cleaning baffle 7.

In more detail, the tube holder baffle 8 incorporates a sealing system adapted to limit, as much as possible, the by-pass in the area adjacent to the walls of the reactor chamber 2; said sealing system is attained with a series of sliding blocks 11, arranged on the periphery of the baffle 8, kept adherent to the internal wall of the reactor with springs 12; the sliding blocks 11 adhere and slide on the internal wall of the reactor body 2.

As stated, the assembly thus constituted, i.e. that which was also defined as the movable scraper 5, has smaller length than the entire length of the reactor body 2C.

Due to such configuration, said scraper 5 is housed inside the chamber 2 by means of longitudinally arranged support guides 12 fixed to the two bottom ends 2A and 2B of said reactor chamber 2.

More precisely, the scraper 5 is reversibly translatable along the axis AA by means of a cylinder 13 and relative stem (not illustrated) connected to said group 5. The group 5 slides on said support guides 12 which are inserted in respective holes 14 made on all the baffles 7 and 8 of the group 5.

In the embodiment, the movement of the movable scraper 5 occurs as stated by means of a piston and cylinder; nevertheless, without departing from the protective scope, such movement can also be actuated by means of an electric actuator.

In accordance with one embodiment, it is observed that all the cleaning baffles 7 comprise - in addition to the plurality of holes 10 of limited size for the passage of the product to be treated and in addition to the aforesaid holes 14 - a series of holes 15 each of which comprising spring-loaded adherence rings 16 that are integral with the cleaning baffle 7 in contact with the relative quartz tube 3 that crosses it. In such a manner, by moving the group 5 (by means of activation of the cylinder 13), each adherence ring 16 scrapes and cleans the relative quartz tube 3. The movable scraper 5 can also correspond, as stated above, with only one sub-chamber 6.

As stated, due to this solution it is possible to remove, only with the movement of the scraper, the deposits/residues from the quartz tubes, avoiding manual cleaning.

That described above constitutes a first cleaning system "self-managed" by the movable scraper 5, upon its reciprocating movement inside the chamber 2; indeed, this occurs by means of the cleaning baffles 7 and the scraping rings 16 which are moved for the cleaning.

In accordance with possible embodiments, the reactor 1 also provides for a second cleaning system, with which the dead areas not reachable by the scraping rings 16 are cleaned.

In more detail and with reference to the figures, it is observed that the exterior of the chamber 2, i.e. the external casing, is equipped with rings of nozzles 18, which traverse the skirt of the chamber and are connected to a fluid source so as to direct a blade jet of water under pressure towards the interior and hit, also following the movement of the group 5, the hidden areas in proximity to the baffles 7 and 8.

Naturally, the aforesaid external cleaning system with pressurized water occurs under vacuum conditions, i.e. with reactor lacking the fluid to be treated.

Preferably, the tubes 3 are parallel to each other and equidistant with respect to the longitudinal extension axis of the reactor chamber 2. More preferably, the tubes 3 are uniformly distributed around said longitudinal extension axis.

### OPERATION OF THE REACTOR AND CLEANING SYSTEM

In operating configuration, the movable scraper is situated in the middle of the reactor: the fluid enters at one end, passes through the first cleaning baffle, specifically through a plurality of its holes made on most of the baffle-disc. It is observed that the interspace 20 defines the only passage for the fluid through said tube holder baffle 8.

Then, the fluid arrives into the first sub-chamber and is forcibly conveyed to pass through the plurality of passage interspaces 20 obtained from the series of jacket segments 9 coaxial with the quartz tubes 3 (the latter extended over the entire length of the cylinder reactor).

The treated fluid then continues through the second cleaning baffle and continues into the adjacent sub-chamber and hence treatment area and so forth until reaching the final area of the chamber 2, from where it is drawn in order to continue into the various lines downstream.

The actuation of the cleaning system for the baffles occurs by alternately translating the movable scraper 5 inside the reactor chamber 2, and possibly by actuating the external cleaning system with blade jets.

In such a manner, in the apparatus 1 for opaque fluid disinfection with ultraviolet emission, at least one portion of the quartz tube is coaxially inserted in a tube jacket of larger size, and the fluid to be treated passes into the space defined between said tube of larger size and said quartz tube.

## Claims

1. Apparatus (1) for opaque fluid disinfection by means of ultraviolet emission, the apparatus comprising:
• a reactor body (2C), in which a cylindrical reactor chamber (2) is defined, closed at the longitudinal ends by a first and a second terminal respectively provided with an inlet port (4A) from which a fluid to be treated enters and with an outlet port (4B) from which the treated fluid exits from the reactor chamber (2);
• at least one ultraviolet light source housed in a tube (3) longitudinally extended inside the reactor chamber (2);
said apparatus (1) being **characterized in that** it comprises, inside the reactor chamber (2):
• a tube holder baffle (8) comprising a sealing system in the area adjacent to the internal walls of the reactor body (2C), and
• a tubular jacket (9) supported by said tube holder baffle (8), said jacket (9) being coaxial with the tube (3) and having larger size with respect to the latter so as to define an annular interspace (20) between the tube (3) and the jacket (9) in a manner such to allow the passage of the fluid to be treated through the tube holder baffle (8), where said interspace (20) defines the only passage of the fluid to be treated through the tube holder baffle (8).

2. Apparatus according to claim 1, wherein said apparatus comprises a movable scraper (5) translatable along the longitudinal extension of the reactor chamber (2), inside the same, said movable scraper (5) comprising at least one pair of cleaning baffles (7).

3. Apparatus according to claim 1 or 2, wherein said sealing system comprises a series of sliding blocks (11), arranged on the periphery of the baffle (8), said sliding blocks being kept adherent to the internal wall of the reactor with springs (12), and adapted to slide on the internal wall of the reactor chamber (2) following the translation of the movable scraper (5).

4. Apparatus according to claim 2 or 3, when dependent on claim 2, wherein each baffle (7) has:
a. a series of holes (10) adapted to allow the passage of the fluid to be treated through said baffle (7),
b. at least one hole (15) for the passage of said tube (3), said hole (15) being associated with a spring-loaded adherence ring (16), which remains in contact with the tube (3) and is adapted to scrape and clean it following the translation of the movable scraper (5).

5. Apparatus according to claim 4, wherein said apparatus comprises a plurality of tube holder baffles (8), each tube holder baffle (8) supporting a respective tubular jacket (9) and dividing a respective sub-chamber (6) delimited by a pair of cleaning baffles (7).

6. Apparatus according to claim 5, wherein the longitudinal extension (L) of the jacket (9) is less than the distance (L1) between a pair of baffles defining the sub-chamber (6).

7. Apparatus according to one of claims 2 to 6, wherein said movable scraper (5) has smaller length than the entire length of the reactor body and is housed inside the chamber (2) by means of longitudinally arranged support guides (19) fixed to the terminals (2A) and (2B) of said reactor body (2C).

8. Apparatus according to one of claims 2 to 7, wherein said movable scraper (5) is reversibly translatable along an axis (AA) of the reactor body (2C), the movement of the movable scraper (5) occurring by means of a cylinder (13) and relative stem connected to the movable scraper (5) or by means of an electric actuator.

9. Apparatus according to one of claims 7 to 8, wherein the movable scraper (5) slides on said support guides (19) which are inserted in respective holes (14) formed on the baffles (7) and on the tube holder baffle(s) (8).

10. Apparatus according to claim 4 or one of claims 5 to 9 when dependent on claim 4, comprising at least three baffles (7) which define a pair of sub-chambers (6), wherein said sub-chambers (6) are connected to each other and divided by the respective baffles (7).

11. Apparatus according to one of the preceding claims, comprising an external washing system comprising a plurality of nozzles (18) which direct a blade jet of water under pressure towards the interior of the reactor chamber (2).

12. Apparatus according to one of the preceding claims, wherein the reactor chamber (2) comprises a tube bundle therein constituted by a plurality of tubes (3) in turn containing a respective germicidal lamp (21), tubes (3) and lamps (21) being fixed.

13. Apparatus according to claim 12, wherein said tubes (3) are parallel to each other and equidistant with respect to the longitudinal extension axis of the reactor chamber (2).

14. Apparatus according to claim 13, wherein said tubes (3) are uniformly distributed around said longitudinal extension axis.

15. Apparatus according to one of the preceding claims, wherein said tube (3) is made of quartz.

16. Apparatus according to one of the preceding claims, wherein said movable scraper (5) can be associated in at least one sub-chamber (6) which identifies a treatment area (17), said sub-chamber (6) being divided into two parts (6A, 6B) by said tube holder baffle (8).

17. Apparatus according to one of the preceding claims, wherein the thickness of said annular interspace (20) is comprised in the interval between 3 and 6 millimeters.

## Patentansprüche

1. Vorrichtung (1) zur Desinfektion von undurchsichtigen Flüssigkeiten mittels UV-Emission, wobei die Vorrichtung Folgendes umfasst:
• einen Reaktorkörper (2C), in dem eine zylindrische Reaktorkammer (2) definiert ist, die an den Längsenden durch einen ersten und einen zweiten Anschluss geschlossen ist, die jeweils mit einer Einlassöffnung (4A) versehen sind, aus der ein zu behandelndes Fluid eintritt und mit einer Auslassöffnung (4B), aus der das behandelte Fluid aus der Reaktorkammer (2) austritt;
• mindestens eine UV-Lichtquelle, die in einem Rohr (3) untergebracht ist, das sich in Längsrichtung innerhalb der Reaktorkammer (2) erstreckt;
wobei die Vorrichtung (1) **dadurch gekennzeichnet ist, dass** sie innerhalb der Reaktorkammer (2) umfasst:
• eine Rohrhalterklappe (8), die ein Dichtungssystem in dem Bereich umfasst, der an die Innenwände des Reaktorkörpers (2C) angrenzt, und
• einen röhrenförmigen Mantel (9), der von der Rohrhalterklappe (8) getragen wird, wobei der Mantel (9) koaxial mit dem Rohr (3) ist und in Bezug auf letzteres größer ist, um einen ringförmigen Zwischenraum (20) zwischen dem Rohr (3) und dem Mantel (9) in einer solchen Weise zu definieren, dass das zu behandelnde Fluid durch die Rohrhalterklappe (8) hindurchtreten kann, wobei der Zwischenraum (20) die einzige Passage der Fluides definiert, die durch die Rohrhalterklappe (8) behandelt werden soll.

2. Vorrichtung nach Anspruch 1, wobei die Vorrichtung einen beweglichen Abstreifer (5) umfasst, der entlang der Längserstreckung der Reaktorkammer (2) innerhalb derselben verschiebbar ist, wobei der bewegliche Schaber (5) mindestens ein Paar Reinigungsklappen (7) umfasst.

3. Vorrichtung nach Anspruch 1 oder 2, wobei das Dichtungssystem eine Reihe von Gleitblöcken (11) umfasst, die an der Peripherie der Klappe (8) angeordnet sind, wobei die Gleitblöcke durch Federn (12) an der Innenwand des Reaktors haftend gehalten werden und angepasst sind, um auf der Innenwand der Reaktorkammer (2) zu gleiten, der Verschiebung des beweglichen Abstreifers (5) folgend.

4. Vorrichtung nach Anspruch 2 oder 3, wenn von Anspruch 2 abhängig, wobei jede Klappe (7) umfasst:
a. eine Reihe von Löchern (10), die geeignet sind, den Durchgang des zu behandelnden Fluides durch die Klappe (7) zu ermöglichen,
b. mindestens ein Loch (15) für den Durchgang des Rohrs (3), wobei das Loch (15) einem federbelasteten Haftring (16) zugeordnet ist, der mit dem Rohr (3) in Kontakt bleibt und zum Abstreifen und folgend der Verschiebung des beweglichen Abstreifers (5) zum Reinigen desselben, geeignet ist.

5. Vorrichtung nach Anspruch 4, wobei die Vorrichtung mehrere Rohrhalterklappen (8) umfasst, wobei jede Rohrhalterklappe (8) einen entsprechenden rohrförmigen Mantel (9) trägt und eine entsprechende Unterkammer (6) unterteilt, die durch ein Paar von Reinigungsklappen (7) begrenzt ist.

6. Vorrichtung nach Anspruch 5, wobei die Längsausdehnung (L) des Mantels (9) kleiner ist als der Abstand (L1) zwischen einem Paar Leitbleche, die die Unterkammer (6) definieren.

7. Vorrichtung nach einem der Ansprüche 2 bis 6, wobei der bewegliche Abstreifer (5) eine geringere Länge als die Gesamtlänge des Reaktorkörpers hat und innerhalb der Kammer (2) mittels in Längsrichtung angeordneter, daran befestigter Stützführungen (19) untergebracht und mit Anschlüssen (2A) und (2B) des Reaktorkörpers (2C) befestigt ist.

8. Vorrichtung nach einem der Ansprüche 2 bis 7, wobei der bewegliche Abstreifer (5) entlang einer Achse (AA) des Reaktorkörpers (2C) reversibel verschiebbar ist, wobei die Bewegung des beweglichen Abstreifers (5) mittels eines Zylinders (13) und einer entsprechenden Stange, die mit dem beweglichen Abstreifer (5) oder mittels eines elektrischen Aktuators, verbunden ist, erfolgst.

9. Vorrichtung nach einem der Ansprüche 7 bis 8, wobei der bewegbare Abstreifer (5) auf den Stützführungen (19) gleitet, die in entsprechende Löcher (14) eingesetzt sind, die an den Ablenkplatten (7) und an der (den) Ablenkplatte(n) der Rohrhalterklappe (8) ausgebildet sind.

10. Vorrichtung nach Anspruch 4 oder einem der Ansprüche 5 bis 9, wenn von Anspruch 4 abhängig, mit mindestens drei Ablenkplatten (7), die ein Paar Unterkammern (6) definieren, wobei die Unterkammern (6) untereinander verbunden und durch die jeweiligen Ablenkplatten (7) geteilt sind.

11. Vorrichtung nach einem der vorangehenden Ansprüche, umfassend ein äußeres Waschsystem, umfassend eine Vielzahl von Düsen (18), die einen blattförmigen unter Druck auf das Innere der Reaktorkammer (2) gerichteten Strahl aufweist.

12. Vorrichtung nach einem der vorangehenden Ansprüche, wobei die Reaktorkammer (2) ein Rohrbündel darin umfasst, das aus einer Vielzahl von Rohren (3) besteht, die wiederum eine jeweilige keimtötende Lampe (21), Rohre (3) und Lampen (21) enthält, die befestigt sind.

13. Vorrichtung nach Anspruch 12, wobei die Rohre (3) parallel zueinander und äquidistant bezüglich der Längserstreckungsachse der Reaktorkammer (2) sind.

14. Vorrichtung nach Anspruch 13, wobei die Rohre (3) gleichmäßig um die Längserstreckungsachse herum verteilt sind.

15. Vorrichtung nach einem der vorangehenden Ansprüche, wobei das Rohr (3) aus Quarz hergestellt ist.

16. Vorrichtung nach einem der vorhergehenden Ansprüche, wobei der bewegbare Abstreifer (5) mindestens einer Unterkammer (6) zuordenbar ist, die einen Behandlungsbereich (17) kennzeichnet, wobei die Unterkammer (6) in zwei Teile (6A, 6B) durch die Rohrhalterklappe (8) geteilt ist.

17. Vorrichtung nach einem der vorangehenden Ansprüche, wobei die Dicke des ringförmigen Zwischenraums (20) im Bereich zwischen 3 und 6 mm liegt.

## Revendications

1. Appareil (1) pour la désinfection de fluide opaque au moyen d'une émission ultraviolette, l'appareil comprenant :
• un corps de réacteur (2C), dans lequel est délimitée une chambre (2) d'un réacteur cylindrique, fermée aux extrémités longitudinales par une première et une deuxième borne respectivement pourvues d'une ouverture d'entrée (4A) par laquelle entre un fluide à traiter et avec une ouverture de sortie (4B) à partir de laquelle le fluide traité sort de la chambre (2) du réacteur ;
• au moins une source de lumière ultraviolette logée dans un tube (3) prolongé longitudinalement à l'intérieur de la chambre (2) du réacteur ;
ledit appareil (1) étant **caractérisé en ce qu'**il comprend, à l'intérieur de la chambre (2) du réacteur :
• un déflecteur (8) porte-tube comprenant un système d'étanchéité dans la zone adjacente aux parois internes du corps (2C) du réacteur , et
• une chemise tubulaire (9) supportée par ledit déflecteur porte-tube (8), ladite chemise (9) étant coaxiale au tube (3) et ayant une dimension plus grande par rapport à ce dernier de manière à définir un espace annulaire (20) entre le tube (3) et la chemise (9) de manière à permettre le passage du fluide à traiter à travers le déflecteur porte-tube (8), dans lequel ledit espace (20) définit le seul passage du fluide à traiter à travers le déflecteur porte-tube (8).

2. Appareil selon la revendication 1, dans lequel ledit appareil comprend un racleur mobile (5) pouvant être translaté le long de l'extension longitudinale de la chambre (2) du réacteur, à l'intérieur de celle-ci, ledit racleur mobile (5) comprenant au moins une paire de déflecteurs de nettoyage (7).

3. Appareil selon la revendication 1 ou 2, dans lequel ledit système d'étanchéité comprend une série de blocs coulissants (11), disposés sur la périphérie du déflecteur (8), lesdits blocs coulissants étant maintenus adhérents à la paroi interne du réacteur avec des ressorts (12), et apte à coulisser sur la paroi interne de la chambre (2) du réacteur suite à la translation du racleur mobile (5).

4. Appareil selon la revendication 2 ou 3, lorsqu'elle dépend de la revendication 2, dans lequel chaque déflecteur (7) a :
a. une série de trous (10) adaptés pour permettre le passage du fluide à traiter à travers ledit déflecteur (7),
b. au moins un trou (15) pour le passage dudit tube (3), ledit trou (15) étant associé à une bague d'adhérence à ressort (16), qui reste en contact avec le tube (3) et est adaptée pour racler et le nettoyer suite à la translation du racleur mobile (5).

5. Appareil selon la revendication 4, dans lequel ledit appareil comprend une pluralité de déflecteurs porte-tube (8), chaque déflecteur porte-tube (8) supportant une chemise tubulaire respective (9) et divisant une sous-chambre respective (6) délimitée par une paire de déflecteur de nettoyage (7).

6. Appareil selon la revendication 5, dans lequel l'extension longitudinale (L) de la chemise (9) est inférieure à la distance (L₁) entre une paire de déflecteurs définissant la sous-chambre (6).

7. Appareil selon l'une des revendications 2 à 6, dans lequel ledit racleur mobile (5) a une longueur inférieure à toute la longueur du corps du réacteur et est logé à l'intérieur de la chambre (2) au moyen de guides de support (19) disposés longitudinalement fixés au bornes (2A) et (2B) dudit corps (2C) du réacteur.

8. Appareil selon l'une des revendications 2 à 7, dans lequel ledit racleur mobile (5) est déplaçable en translation réversible le long d'un axe (AA) du corps (2C) du réacteur, le déplacement du racleur mobile (5) s'effectuant au moyen d'un vérin (13) et tige relative reliée au racleur mobile (5) ou au moyen d'un actionneur électrique.

9. Appareil selon l'une des revendications 7 à 8, dans lequel le racleur mobile (5) coulisse sur lesdits guides de support (19) qui sont insérés dans des trous respectifs (14) ménagés sur les déflecteurs (7) et sur le ou les déflecteurs porte-tubes (8).

10. Appareil selon la revendication 4 ou l'une des revendications 5 à 9 lorsqu'elles dépendent de la revendication 4, comprenant au moins trois déflecteurs (7) qui définissent une paire de sous-chambres (6), dans lequel lesdites sous-chambres (6) sont reliées chacune l'autre et divisées par les déflecteurs respectives (7).

11. Appareil selon l'une des revendications précédentes, comprenant un système de lavage externe comprenant une pluralité de buses (18) qui dirigent un jet de lame d'eau sous pression vers l'intérieur de la chambre (2) du réacteur.

12. Appareil selon l'une des revendications précédentes, dans lequel la chambre (2) du réacteur comprend un faisceau de tubes constitué par une pluralité de tubes (3) contenant à leur tour une lampe germicide respective (21), des tubes (3) et des lampes (21) étant fixées.

13. Appareil selon la revendication 12, dans lequel lesdits tubes (3) sont parallèles entre eux et équidistants par rapport à l'axe d'extension longitudinal de la chambre (2) du réacteur.

14. Appareil selon la revendication 13, dans lequel lesdits tubes (3) sont uniformément répartis autour dudit axe d'extension longitudinal.

15. Appareil selon l'une des revendications précédentes, dans lequel ledit tube (3) est en quartz.

16. Appareil selon l'une des revendications précédentes, dans lequel ledit racleur mobile (5) peut être associé à au moins une sous-chambre (6) qui identifie une zone de traitement (17), ladite sous-chambre (6) étant divisée en deux parties (6A, 6B) par ledit déflecteur porte-tube (8).

17. Appareil selon l'une des revendications précédentes, dans lequel l'épaisseur dudit espace annulaire (20) est comprise dans l'intervalle entre 3 et 6 millimètres.
